Europäisches Patentamt

European Patent Office

Office européen des brevets

(19)

(11) **EP 0 733 401 B1**

(12) **EUROPÄISCHE PATENTSCHRIFT**

(45) Veröffentlichungstag und Bekanntmachung des Hinweises auf die Patenterteilung:
**05.12.2001 Patentblatt 2001/49**

(51) Int Cl.7: **B01J 10/00**, B01J 3/00, B01J 19/26, B01J 19/00

(21) Anmeldenummer: **96104305.6**

(22) Anmeldetag: **19.03.1996**

(54) **Verfahren zur Durchführung von Gas/Flüssigreaktionen unter Vermeidung einer zusammenhängenden Gasphase**

Process for the treatment of the exhaust gas from the gasification of carbonaceous material

Procédé de traitement des gaz de fumées provenant de la gazéification de matières carbonées

(84) Benannte Vertragsstaaten:
**BE DE FR GB NL**

(30) Priorität: **24.03.1995 DE 19510890**
**23.11.1995 DE 19543636**

(43) Veröffentlichungstag der Anmeldung:
**25.09.1996 Patentblatt 1996/39**

(73) Patentinhaber: **BASF AKTIENGESELLSCHAFT**
**67056 Ludwigshafen (DE)**

(72) Erfinder:
- **Bittins, Klaus, Dr.**
  **67227 Frankenthal (DE)**
- **Heider, Marc, Dr.**
  **67433 Neustadt (DE)**
- **Schmidt-Radde, Martin, Dr.**
  **67259 Beindersheim (DE)**
- **Kellenbenz, Jochen, Dr.**
  **67098 Bad Dürkheim (DE)**
- **Wagner, Kurt Josef**
  **67354 Römerberg (DE)**
- **Zehner, Peter, Dr.**
  **67071 Ludwigshafen (DE)**
- **Berg, Stefan, Dr.**
  **67227 Frankenthal (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:
DE-A- 3 206 661        DE-B- 2 421 407
FR-A- 2 395 977        GB-A- 833 664
GB-A- 1 045 627

- **INDUSTRIAL AND ENGINEERING CHEMISTRY, PROCESS DESIGN AND DEVELOPMENT, Bd. 1, Nr. 2, 1.April 1962, Seiten 137-141, XP000571521 NEDWICK J J: "LIQUID PHASE PROCESS FOR ACETYLENE REACTIONS"**
- **INDUSTRIAL AND ENGINEERING CHEMISTRY, Bd. 40, Nr. 7, 1.Juli 1948, Seiten 1171-1177, XP000572476 HANFORD W E ET AL: "ACETYLENE CHEMISTRY"**

**Beschreibung**

Die vorliegende Erfindung betrifft ein Verfahren zur Durchführung von Gas/Flüssig-Reaktionen unter Vermeidung einer zusammenhängenden Gasphase.

Aus der DE-A-32 06 661 ist ein Verfahren zur Vermeidung einer zusammenhängenden Gasphase mittels eines Treibstrahls bekannt. Fällt dieser Treibstrahl aus, so führt dies zur Separation von Gas und Flüssigkeit. Die entstehende Gasphase muß folglich in möglichst kurzer Zeit durch Inertisierung (Einpressen von Inertgas) aus dem Explosionsbereich geführt werden.

Aus der DE-A-24 21 407 ist ein Verfahren zur Vermeidung einer zusammenhängenden Gasphase mittels einer Regelung der Gaszufuhr auf Werte unterhalb der Sättigungsgrenze als zusätzliches Sicherheitselement hinzugefügt.

Das in der DE-A-24 21 407 beschriebene Verfahren eignet sich in der beschriebenen Ausführung lediglich für kontinuierliche Prozesse. Sofern diskontinuierliche Umsetzungen, z.B. aufgrund der Forderung nach Vollumsatz erforderlich sind, scheidet dieses Verfahren aus. Das in der DE-A-32 06 601 beschriebene diskontinuierliche Verfahren kann lediglich für Systeme eingesetzt werden, die nur eine geringe Volumenzunahme im Verlauf der Umsetzung aufweisen. Dies ist auf den vor Reaktionsbeginn fest vorgegebenen Inertgas hold-up zurückzuführen, der der maximal möglichen Volumenzunahme entspricht.

Acetylen kann für eine Vielzahl von Produkten industriell genutzt werden (Ullmann Vol. A1, 5. Auflage, Seite 101 bis 105). Der Umgang mit Acetylen erfordert besondere Sicherheitsvorkehrungen, da gasförmiges Acetylen unter Druck zur Zersetzung neigt (Chem.-Ing.-Tech. 66 (1994) 1389 bis 1392; S.A. Miller, Acetylene, Ernest Benn Limited 1965, Vol. 1, Seite 485 bis 506). Vor allem die für die verschiedenen Reaktionen eingesetzten Katalysatoren initiieren eine solche Zersetzung, falls sie sich im Gasraum der eingesetzten Reaktoren ablagern.

In Ind. Eng. Chem. Proc. Design. & Dev. 1 (1962) 137 bis 141 wird empfohlen, das Acetylen zunächst in einem separaten Sättiger in dem umzusetzenden Stoff und einem geeigneten Lösungsmittel zu lösen und diese Lösung dann in einem Reaktor umzusetzen. Dabei muß der Druck so groß gewählt werden, daß eine Gasphase im Reaktor ausgeschlossen werden kann, was die Verwendung geeigneter Hochdruckapparate zur Folge hat. Außerdem müssen bei diesem Verfahren geeignete Lösungsmittel verwendet werden, deren Abtrennung aufwendig ist. Falls das dort beschriebene Verfahren kontinuierlich arbeitet, ist die dem Reaktor zuzuführende Acetylenmenge annähernd konstant und eine Acetylen-Gasphase im Reaktor kann aufgrund evtl. zu großer Acetylenzufuhr vermieden werden.

Bei der diskontinuierlichen Umsetzung von Acetylen ist diese Vorgehensweise jedoch äußerst schwierig, da die reaktionsbedingte Acetylenaufnahme zeitlich nicht konstant ist, die Acetylenkonzentration im Reaktor aber nicht kontrolliert werden kann. Durch das unterschiedliche Lösungsvermögen von Acetylen in Ausgangs- und Endprodukten ist darüberhinaus die im Reaktorinhalt lösliche Acetylenmenge ständigen Änderungen unterworfen. Außerdem hat das beschriebene Verfahren für diskontinuierliche Umsetzungen den Nachteil, daß große Flüssigkeitsmengen im Kreis gefahren werden müssen.

Diskontinuierliche Umsetzungen mit Acetylen unter Druck werden daher in Anwesenheit einer Acetylen-Gasphase durchgeführt (z.B. Ind. Eng. Chem. 40 (1948) 1171 bis 1177). Aufgrund der oben beschriebenen Zersetzungsgefahr von verdichtetem Acetylen bedarf ein solches Verfahren eines erheblichen Sicherheitsaufwands, wodurch aber Zersetzungen nicht völlig ausgeschlossen werden können. Diskontinuierliche Umsetzungen mit Acetylen unter Druck sind aber vor allem dann erforderlich, wenn ein nahezu vollständiger Umsatz des Edukts erzielt werden soll. Dies kann z.B. dann der Fall sein, wenn Edukt und Produkt nicht oder nur mit sehr hohem Aufwand getrennt werden können.

GB 1,045,627 beschreibt ein diskontinuierliches Verfahren zur Herstellung von N-vinyl-$\alpha$-pyrrolidon aus $\alpha$-Pyrrolidon und Acetylen unter Druck. Durch Einsatz eines Rührers wird dabei eine sogenannte Gas/Flüssig-Emulsion erhalten, d.h. es wird oberhalb der Sättigungsgrenze von 100 % gearbeitet. Es bestand daher die Aufgabe, den zuvor genannten Nachteilen abzuhelfen, insbesondere ein sicheres Verfahren für diskontinuierliche Umsetzungen mit Acetylen unter Druck zu finden, das für eine Vielzahl von Produkten und Reaktionen geeignet ist.

Demgemäß wurde ein neues und verbessertes Verfahren zur Durchführung von Gas/Flüssigreaktionen bei Temperaturen von (-50) bis 300 °C und einem Druck von 0,1 bis 100 bar gefunden, wobei man in Abwesenheit einer zusammenhängenden Gasphase arbeitet, das dadurch gekennzeichnet, daß man einen Reaktor vollständig mit Reaktionsansatz füllt, dem Reaktor kontinuierlich einen Überlauf als Austragsstrom entnimmt, diesen in einem Ausgleichsbehälter entspannt und in den Reaktor zurückführt, wobei man den gasförmigen Reaktanten diskontinuierlich in die flüssige Phase bis zu einem Sättigungsgrad von 5 bis 100 % einleitet und die Gaskonzentration in dem Reaktor aus dem bei der Entspannung anfallenden Abgasstrom, dem Flüssigkeitsstrom und dem Druckabfall ermittelt, sowie als Spezialfall ein Verfahren zur diskontinuierlichen Umsetzung von Acetylen in flüssiger Phase bei Temperaturen von 0 bis 300°C und Drücken von 2 bis 30 bar, in dem man in Abwesenheit einer Gasphase Acetylen isobar bis zu einem Sättigungsgrad ven 5 bis 100% in die flüssige Phase einleitet.

Die Vermeidung einer zusammenhängenden Gasphase kann sowohl aufgrund unerwünschter Nebenreaktionen in der Gasphase, als auch aufgrund eines hohen Gefährdungspotentials zwingend erforderlich oder wünschenswert sein. Das Gefährdungspotential resultiert aus der Möglichkeit eines spontanen Zerfalls in der Gasphase, oder der sehr heftigen (z.B. explosionsartigen) Reaktion des gasförmigen Stoffes mit im Reaktorsystem vorhandenen Reaktionspartnern.

Das erfindungsgemäße Verfahren kann wie folgt durchgeführt werden:

Bei Temperaturen von (-50) bis 300°C und einem Druck von 0,1 bis 100 bar wird der flüssige Reaktand mit dem gasförmigen Reaktionspartner, unter Ausschluß einer zusammenhängenden Gasphase im Reaktor oder einer Reaktorkaskade umgesetzt.

Als zusammenhängende Gasphase werden Gasräume innerhalb des Reaktionsraums verstanden, deren Größe über einzelne diskrete Blasen oder Bläschen hinausgeht.

Am höchsten Punkt eines Reaktors (z.B. eines jeden Reaktors einer Kaskade) wird der Produktstrom in der Regel kontinuierlich abgezogen. Eventuell auftretende Gasblasen, die am Reaktorkopf ankommen können, werden durch den Flüssigkeitsstrom in der Regel sofort aus dem Reaktorsystem ausgeschleußt.

Nach Verlassen des Reaktors werden die Betriebsparameter - z.B. in einem anschließenden Phasenscheider - so gewählt, daß sich dieser Bereich in einem sicheren Zustand befindet. Dies kann durch Absenkung von Druck und/oder Temperatur und/oder durch Inertisierung mit einem Inertgas wie Stickstoff, Argon, Kohlendioxid, Wasserdampf, bevorzugt Stickstoff, Kohlendioxid, besonders bevorzugt Stickstoff erfolgen.

Zusätzlich zu diesem Vorgehen zur Vermeidung einer zusammenhängenden Gasphase können zwei weitere ergänzende Maßnahmen - alleine oder in Kombination - ergriffen werden:

a) Die Regelung der Gaskonzentration (Sättigung) im Reaktoraustrag wird in der Regel auf 5 bis 100%, bevorzugt auf Werte unterhalb der maximalen Gaslöslichkeit wie 10 bis 95%, besonders bevorzugt 20 bis 80%, insbesondere 30 bis 80%, speziell 40 bis 70% gesteuert. Dies verhindert bereits die Entstehung freier Gasblasen in der folglich untersättigten Flüssigkeit, so daß der Reaktorkopf in der Regel blasenfrei ist.

Die Ermittlung der Konzentration der gasförmigen Komponente und damit die Regelung der Gaszufuhr erfolgt durch die im folgenden beschriebene einfache Bestimmung des Abgasstroms nach einer Entspannung. Der bei der Entspannung anfallende Abgasstrom wird durch die Größe des Austragstroms, die darin gelöste Gasmenge und dem Druckabfall bei der Entspannung bestimmt. Aus dem Verhältnis von Abgasstrom und Flüssigkeitsstrom kann bei konstantem Druckabfall die Gaskonzentration in der Austragsleitung ermittelt werden. Falls der Reaktor aufgrund der großen inneren und äußeren Zirkulationsströme als ideal durchmischt betrachtet werden kann, ist damit auch die Gaskonzentration im Reaktor selbst bekannt und kann auf einen vorgegebenen Sollwert, unterhalb der Sättigungsgrenze geregelt werden.

b) Bei geeigneter Anordnung einer Strahldüse am Reaktorkopf, vorzugsweise im Reaktordeckel, kann der Flüssigkeitsimpuls zur Redispergierung des Gases verwendet werden. Sollten größere Blasen den Reaktorkopf erreichen, werden diese wieder zerteilt. Bei diesem Vorgehen kann sich eine geschlossene Gasphase selbst bei Ausfall der kontinuierlichen Durchströmung des Reaktors erst bei hohen Gasbelastungen ausbilden.

Um auch bei diskontinuierlichen Umsetzungen die erforderliche kontinuierliche Durchströmung des Reaktionsraums realisieren zu können, umfaßt das Reaktorsystem im diskontinuierlichen Fall neben dem Reaktor (der Reaktorkaskade) in der Regel einen Ausgleichsbehälter, der als Phasenscheider arbeitet und durch geeignete Maßnahmen (Druck-, Temperaturabsenkung, Inertisierung) abzusichern ist. Dieser kann eine Volumenzunahme im Verlauf der Reaktion kompensieren. Sofern bei den durchgeführten Reaktionen Vollumsatz angestrebt wird, ist der Ausgleichsbehälter vorzugsweise durch Einbauten oder Unterteilung zu kaskadieren. Aus dem Ausgleichsbehälter wird kontinuierlich ein Teilstrom in den Reaktionsraum zurückgeführt. Die Menge des aus dem Ausgleichsbehälter pro Zeiteinheit zurückgeführten Stroms ist unkritisch. Sie sollte an die Reaktionsgeschwindigkeit angepaßt werden und ca. die 0,1- bis 10-fache Menge des Reaktorinhalts bezogen auf die gesamte Reaktionszeit betragen.

Die Reaktion kann bis zu einem beliebigen Umsatz durchgeführt werden, der z.B. durch kontinuierliche Analyse des Austragstroms verfolgt werden kann. Auch die vollständige Umsetzung von Reaktorinhalt und Ausgleichsbehälter im diskontinuierlichen Fall ist möglich und häufig erwünscht, um spätere Trennprobleme zu vermeiden.

Zur Verdichtung und Dispergierung des Gases kann eine Düse eingesetzt werden. Diese sorgt für eine sehr feine Verteilung der Gasblasen und damit für eine große Phasengrenzfläche im Bereich hoher Turbulenz, wodurch das Gas rasch in der Flüssigkeit gelöst wird. Die Düse kann wahlweise am Boden, oder am Kopf des Reaktors angeordnet sein, wobei im letzteren Fall vorzugsweise ein konzentrisches Einsteckrohr eingebaut werden sollte. Die Anordnung der Düse im höchsten Punkt des Reaktors besitzt den Vorteil, daß im Stillstand keine Flüssigkeit in den gasführenden Teil der Düse zurücksteigen kann. Die Verwendung einer selbstansaugenden Düse stellt eine zusätzliche, integrierte Sicherheitseinrichtung dar, da bei Ausfall der Pumpe und damit des Flüssigkeitstreibstrahls, z.B. im Störfall, kein Gas mehr angesaugt wird.

Als weitere Sicherheitseinrichtung kann ein geeigneter Blasendetektor an der Austragsleitung vor einer möglichen Druckentlastung installiert werden.

Das erfindungsgemäße Verfahren ist für eine Vielzahl von Gas/Flüssigreaktionen geeignet. Beispiele für gasförmige Reaktanden, die ein hohes Gefährdungspotential aufweisen und damit insbesondere in Frage kommen, sind:

Acetylen und höhere Homologe; Ketene; Ethylenoxid, Propylenoxid und höhere Homologe; Acrolein; Sauerstoff; Chlor; Chlordioxid; Hydrazin; Stickstoffwasserstoffsäure.

Bei Verwendung des geschilderten integrierten Reaktorsicherheitskonzepts kann ein sprunghafter Druck- und Temperaturanstieg zuverlässig vermieden werden. Dies führt zu erhöhter Betriebssicherheit, kürzeren Stillstandszeiten und verminderten Produktverlusten. Verglichen mit eigensicheren Reaktionssystemen umfaßt das erfindungsgemäße Verfahren ausschließlich einfache Apparate, die nicht mehr für den Explosionsdruck, sondern lediglich für eine an den Betriebsdruck angepaßte Druckstufe ausgelegt werden müssen. Hieraus ergeben sich Einsparungen bei den Investitionskosten. Für den Fall der Umsetzung mit Acetylen kann erfindungsgemäß wie folgt vorgegangen werden:

In einem abgeschlossenem Reaktionsraum (z.B. einem Reaktor) kann in Abwesenheit einer Gasphase Acetylen, bevorzugt fein bis feinst verteilt in die flüssige Reaktionsmischung bei Temperaturen von 0 bis 300°C, bevorzugt 20 bis 200°C isobar, also konstantem oder nahezu konstantem Druck, wobei die Druckschwankungen 0 bis 20%, bevorzugt 0 bis 10%, besonders bevorzugt 0 bis 5%, insbesondere 0 bis 2% des eingestellten Druckes betragen, eingeleitet werden. Der Druck kann in der Regel 2 bis 30 bar, bevorzugt 10 bis 25 bar, besonders bevorzugt 15 bis 20 bar betragen.

In einer bevorzugten Ausführungsform wird ein Strahldüsenreaktor vollständig mit Reaktionsansatz gefüllt und mit Hilfe einer Pumpe aus einem Ausgleichsbehälter ein ständiger Flüssigkeitsstrom in den Reaktor zurückgeführt. Der dadurch und durch die reaktionsbedingte Volumenzunahme verursachte Überlauf wird am Kopf des Reaktors mit Hilfe eines Regelventils, welches den Reaktordruck konstant hält, in den Ausgleichsbehälter entspannt und seine Menge gemessen. Die Menge des dabei entweichenden Acetylens wird ebenfalls gemessen, wodurch man eine direkte Information über die Sättigung des Reaktorinhalts mit Acetylen erhält. Über die Formel

$$\text{Verhältnis} = \text{Menge Acetylen im Überlauf} / \text{Menge Überlauf}$$

wird ein Verhältniswert ermittelt, der einem Sollwert entsprechen sollte. Liegt das ermittelte Verhältnis über dem Sollwert, wird die Acetylenzufuhr gedrosselt, liegt es darunter, wird sie erhöht. Mit dem Fachmann bekannten Methoden kann in separaten Versuchen die maximale Löslichkeit von Acetylen in den Ausgangsstoffen und Reaktionsprodukten unter Reaktionsbedingungen bestimmt werden, woraus der Sollwert abgeleitet werden kann.

Mit einer Pumpe wird ein Flüssigkeitsstrahl erzeugt, der das verdichtete Acetylen in der Strahldüse ansaugt und so schnell in die Flüssigkeit einlöst, daß keine Acetylen-Gasphase, die zersetzlich wäre, im Reaktor auftritt.

Als Reaktor kann ein Strahldüsenreaktor, wie er in Ullmann, Vol B4, S. 297 bis 307 beschrieben wird, eingesetzt werden. Es ist dabei darauf zu achten, daß sich in dem Reaktor keine Gasphase aufbauen kann. Die Sättigung des Reaktorinhalts mit Acetylen in der flüssigen Phase kann in der Regel 5 bis 100%, bevorzugt 10 bis 95%, besonders bevorzugt 30 bis 80%, insbesondere 40 bis 70% über den gesamten Reaktionsverlauf konstant gehalten werden. Die Menge des aus dem Ausgleichsbehälter in den Reaktor zurückgeführten Überlaufs pro Zeiteinheit ist nicht kritisch und kann das 0,01 bis 2-fache des Reaktorinhalts pro Stunde betragen. Bevorzugt ist hier das 0,1 bis 1-fache des Reaktorinhalts pro Stunde. Die Reaktion kann bis zu einem beliebigen Umsatz an Edukt durchgeführt werden, was z.B. durch ständige Analyse des Überlaufs verfolgt werden kann. Auch die vollständige Umsetzung von Reaktorinhalt und Ausgleichsbehälter ist möglich und häufig erwünscht, um spätere Trennprobleme zu vermeiden.

Dieses Verfahren ist für eine Vielzahl von Reaktionen mit Acetylen wie z.B. die Vinylierung [Liebigs Anm. Chem. 601 (1956) 81-138] (Umsetzung acider Verbindungen mit Acetylen zu Vinylverbindungen), die Ethinylierung [W. Reppe, Neue Entwicklungen auf dem Gebiet des Acetylens und Kohlenoxyds, Springer 1949, S. 23-66.] (Umsetzung von Acetylen mit Carbonylverbindungen zu Alkoholen) oder die Carbonylierung [W. Reppe, Neue Entwicklungen auf dem Gebiet des Acetylens und Kohlenoxyds, Springer 1949, S. 94-126] (Umsetzung von Acetylen mit Kohlenoxid und Nucleophilen zu Carbonsäurederivaten) geeignet, bevorzugt für Vinylierungen.

Beispiele

Beispiele 1 bis 8

Ein 6-1-Strahldüsenreaktor wurde hydraulisch mit dem zu vinylierenden Produkt, in dem der Katalysator (Kaliumhydroxid) gelöst war, gefüllt und die in der Tabelle angegebenen Werte für Druck und Temperatur eingestellt. Mit Hilfe einer Spaltrohrmotorpumpe wurde ein Treibstrahl vo ca. 1200 l/h für die Strahldüse erzeugt, mit einer Membranpumpe wurde ein konstanter Mengenstrom aus dem Ausgleichsbehälter in den Reaktor zurückgeführt. Die Acetylenzufuhr wurde über eine Regelung, in die die Austragsmenge und die Abgasmenge einging, eingestellt. Durch stündliche Probenahme wurde der Vinylierungsverlauf verfolgt. Nach beendeter Reaktion wurde der Reaktorinhalt abgekühlt,

entspannt und aufgearbeitet.

Tabelle

| Nr. | Produkt | Druck [bar] | Temperatur [°C] | Laufzeit [h] | Acetylen-zufuhr [l/h] | Acetylen-abgas [l/h] | Sollwert [l/kg] | Rückfüh-rung [kg/h] | Umsatz [%] |
|---|---|---|---|---|---|---|---|---|---|
| 1 | n-Butanol | 18 | 160 | 22 | 390 | 28 | 10 | 4 | 99,7 |
| 2 | Cyclohexanol | 18 | 160 | 14 | 450 | 53 | 10 | 5 | 99,6 |
| 3 | Ethylenglykol | 20 | 160 | 7 | 490 | 20 | 20 | 1 | 59,0 |
| 4 | Butandiol | 19,5 | 160 | 31 | 400 | 55 | 10 | 5,3 | 92,5 |
| 5 | Imidazol | 18 | 160 | 36 | 340 | 36 | 8 | 4 | 87,1 |
| 6 | Diethylen-glykol | 18 | 160 | 46 | 290 | 30 | 7 | 4 | 100 |
| 7 | Cyclohexandi-methanol | 18 | 160 | 15 | 400 | 25 | 5 | 4 | 68,0 |
| 8 | Methyltriethy-lenglykol | 18 | 160 | 10 | 330 | 30 | 10 | 4 | 65,1 |

EP 0 733 401 B1

Beispiel 9

Kontinuierliche Oxidation von Propionaldehyd

In einer dreistufigen Reaktorkaskade bestehend aus zwei Strahldüsenreaktoren und einem Verweilzeitbehälter wurde Propionaldehyd mit reinem Sauerstoff zu Propionsäure oxidiert. Das Reaktorvolumen becrug jeweils 2 1. Die Strahldüsenreaktoren waren mit einer Zweistoffdüse im Reaktordeckel und einem konzentrischen Einsteckrohr ausgerüstet. Die Austragsleitung (Überlauf) der übereinander angeordneten Reaktoren befand sich im höchsten Punkt des Reaktors. Der Produktstrom wurde vor dem Phasenscheider entspannt, der mit Stickstoff inertisiert wurde. Der Treibstrahl der Zweistoffdüse wurde mit einer Kreiselpumpe erzeugt. Die kontinuierliche Zufuhr des Propionaldehyds von 1 l/h erfolgte mit einer Kolbenpumpe. Bei einer Temperatur von 60°C und einem Reaktordruck von 5 bar wurde ein Umsatz von über 98% erreicht.

**Patentansprüche**

1. Verfahren zur Durchführung von Gas/Flüssigreaktionen bei Temperaturen von (-50°C) bis 300°C und einem Druck von 0,1 bis 100 bar in Abwesenheit einer zusammenhängenden Gasphase, **dadurch gekennzeichnet, daß** man einen Reaktor vollständig mit Reaktionsansatz füllt, dem Reaktor kontinuierlich einen Überlauf als Austragsstrom entnimmt, diesen in einem Ausgleichsbehälter entspannt und in den Reaktor zurückführt, wobei man den gasförmigen Reaktanten diskontinuierlich in die flüssige Phase bis zu einem Sättigungsgrad von 5 bis 100 % einleitet und die Gaskonzentration in dem Reaktor aus dem bei der Entspannung anfallenden Abgasstrom, dem Flüssigkeitsstrom und dem Druckabfall ermittelt.

2. Verfahren zur Durchführung von Gas/Flüssigreaktionen nach Anspruch 1, **dadurch gekennzeichnet, daß** man Acetylen bei Temperaturen von 0 bis 300°C und einem Druck von 2 bis 30 bar einleitet.

3. Verfahren zur Durchführung von Gas/Flussigreaktionen nach den Ansprüchen 1 und 2, **dadurch gekennzeichnet, daß** man die Umsetzung bei einen Sättigungsgrad von 10 bis 95% durchführt.

4. Verfahren zur Durchführung von Gas/Flüssigreaktionen nach den Ansprüchen 1 bis 3, **dadurch gekennzeichnet, daß** man die Umsetzung bei einen Sättigungsgrad von 30 bis 80% durchführt.

5. Verfahren zur Durchführung von Gas/Flüssigreaktionen nach den Ansprüchen 1 bis 4, **dadurch gekennzeichnet, daß** man die Umsetzung bei einen Sättigungsgrad von 40 bis 70% durchführt.

6. verfahren zur Durchführung von Gas/Flüssigreaktionen nach den Ansprüchen 1 bis 5, **dadurch gekennzeichnet, daß** man die Umsetzung bei Drücken von 10 bis 25 bar durchführt.

7. Verfahren zur Durchführung von Gas/Flüssigreaktionen nach den Ansprüchen 1 bis 6, **dadurch gekennzeichnet, daß** man die Umsetzung bei Drücken von 15 bis 20 bar durchführt.

8. Verfahren zur Durchführung von Gas/Flüssigreaktionen nach den Ansprüchen 1 bis 7, **dadurch gekennzeichnet, daß** man die Umsetzung bei Temperaturen von 20 bis 200°C durchführt.

9. Verfahren zur Durchführung von Gas/Flüssigreaktionen nach den Ansprüchen 1 bis 8, **dadurch gekennzeichnet, daß** man als Umsetzungen Vinylierungen, Ethinylierungen oder Carbonylierungen durchführt.

10. Verfahren zur Durchführung von Gas/Flüssigreaktionen nach den Ansprüchen 1 bis 9, **dadurch gekennzeichnet, daß** man als Umsetzungen Vinylierungen durchführt.

**Claims**

1. A process for carrying out a gas/liquid reaction at from (-50) to 300°C and from 0.1 to 100 bar in the absence of a continuous gas phase, wherein a reactor is completely filled with a reaction batch, an overflow is continuously removed from the reactor as a discharge stream, the latter is let down in a compensation container and is recycled to the reactor, the gaseous reactant being introduced batchwise into the liquid phase to a degree of saturation of from 5 to 100%, and the gas concentration in the reactor being determined from the exit gas stream obtained or let down, the liquid stream and the pressure drop.

2. A process for carrying out a gas/liquid reaction as claimed in claim 1, wherein acetylene is introduced at from 0 to 300°C and from 2 to 30 bar.

3. A process for carrying out a gas/liquid reaction as claimed in claim 1 or 2, wherein the reaction is carried out at a degree of saturation of from 10 to 95%.

4. A process for carrying out a gas/liquid reaction as claimed in any of claims 1 to 3, wherein the reaction is carried out at a degree of saturation of from 30 to 80%.

5. A process for carrying out a gas/liquid reaction as claimed in any of claims 1 to 4, wherein the reaction is carried out at a degree of saturation of from 40 to 70%.

6. A process for carrying out a gas/liquid reaction as claimed in any of claims 1 to 5, wherein the reaction is carried out at from 10 to 25 bar.

7. A process for carrying out a gas/liquid reaction as claimed in any of claims 1 to 6, wherein the reaction is carried out at from 15 to 20 bar.

8. A process for carrying out a gas/liquid reaction as claimed in any of claims 1 to 7, wherein the reaction is carried out at from 20 to 200°C.

9. A process for carrying out a gas/liquid reaction as claimed in any of claims 1 to 8, wherein the reaction carried out is a vinylation, an ethynylation or a carbonylation.

10. A process for carrying out a gas/liquid reaction as claimed in any of claims 1 to 9, wherein the reaction carried out is a vinylation.

**Revendications**

1. Procédé pour l'exécution de réactions gaz/liquide à des températures de (-50°C) à 300°C et sous une pression de 0,1 à 100 bar, en l'absence d'une phase gazeuse continue, **caractérisé en ce que** l'on remplit totalement un réacteur de la charge réactionnelle, que l'on soutire en continu du réacteur un trop-plein en tant que courant de sortie, que l'on détend celui-ci dans un réservoir de compensation et qu'on le renvoie dans le réacteur, les réactifs gazeux étant introduits en mode discontinu dans la phase liquide jusqu'à un degré de saturation de 5 à 100% et la concentration de gaz dans le réacteur étant déterminée sur base du courant de gaz effluent produit par la détente, du débit de liquide et de la perte de charge.

2. Procédé pour l'exécution de réactions gaz/liquide selon la revendication 1, **caractérisé en ce que** l'on injecte de l'acétylène à des températures de 0 à 300°C et une pression de 2 à 30 bar.

3. Procédé pour l'exécution de réactions gaz/liquide selon les revendications 1 et 2, **caractérisé en ce que** l'on entreprend la réaction à un degré de saturation de 10 à 95%.

4. Procédé pour l'exécution de réactions gaz/liquide selon les revendications 1 à 3, **caractérisé en ce que** l'on entreprend la réaction à un degré de saturation de 30 à 80%.

5. Procédé pour l'exécution de réactions gaz/liquide selon les revendications 1 à 4, **caractérisé en ce que** l'on entreprend la réaction à un degré de saturation de 40 à 70%.

6. Procédé pour l'exécution de réactions gaz/liquide selon les revendications 1 à 5, **caractérisé en ce que** l'on entreprend la réaction à des pressions de 10 à 25 bar.

7. Procédé pour l'exécution de réactions gaz/liquide selon les revendications 1 à 6, **caractérisé en ce que** l'on entreprend la réaction à des pressions de 15 à 20 bar.

8. Procédé pour l'exécution de réactions gaz/liquide selon les revendications 1 à 7, **caractérisé en ce que** l'on entreprend la réaction à des températures de 20 à 200°C.

9. Procédé pour l'exécution de réactions gaz/liquide selon les revendications 1 à 8, **caractérisé en ce que** l'on entreprend, comme réactions, des vinylations, des éthynylations ou des carbonylations.

10. Procédé pour l'exécution de réactions gaz/liquide selon les revendications 1 à 9, **caractérisé en ce que** l'on entreprend comme réactions des vinylations.